# EUROPEAN PATENT APPLICATION

(11) **EP 4 702 987 A1**
(43) Date of publication of application: **04.03.2026**
(21) Application number: 24797185.6
(22) Date of filing: 26.04.2024
(51) Int. Cl.: A61K 38/36, A61K 39/395, A61P 7/04, A61P 43/00

(54) **COMBINED USE OF BISPECIFIC ANTIBODY BINDING TO ACTIVATED COAGULATION FACTOR IX AND COAGULATION FACTOR X WITH BLOOD COAGULATION FACTOR X FOR TREATING SUBJECT WITH HEMOPHILIA A**

(30) Priority: 28.04.2023 JP 2023074020
(71) Applicant: Public University Corporation Nara Medical University, Nara 634-8521 (JP); KM Biologics Co., Ltd., Kumamoto-shi, Kumamoto 860-8568 (JP)
(72) Inventor: NOGAMI, Keiji, Kashihara-shi, Nara 634-8521 (JP); NAKAJIMA, Yuto, Kashihara-shi, Nara 634-8521 (JP); SHIMIZU, Kazuki, Kashihara-shi, Nara 634-8521 (JP); TAKAMI, Eisuke, Kikuchi-shi, Kumamoto 869-1298 (JP); NAKANO, Hirotoshi, Kikuchi-shi, Kumamoto 869-1298 (JP)
(74) Representative: Mewburn Ellis LLP
(86) International application number: PCT/JP2024/016461
(87) International publication number: WO 2024/225440

(57) **Abstract**

Provided is a pharmaceutical composition comprising blood coagulation Factor X for treating a subject with hemophilia A. The present invention relates to a pharmaceutical composition comprising blood coagulation Factor X for treating a subject with hemophilia A, wherein the blood coagulation Factor X is used in combination with a bispecific antibody binding to activated blood coagulation Factor IX and blood coagulation Factor X.

## Description

### TECHNICAL FIELD

### RELATED APPLICATIONS

This application claims the benefit of priority from Japanese Patent Application No. 2023-074020, filed on April 28, 2023, with the Japan Patent Office. The priority application is incorporated herein by reference in its entirety.

The present invention relates to a pharmaceutical composition comprising blood coagulation Factor X for treating a subject with hemophilia A, wherein the blood coagulation Factor X is used in combination with a bispecific antibody that binds to activated blood coagulation Factor IX and blood coagulation Factor X (hereinafter referred to as "FIXa/FX bispecific antibody").

### BACKGROUND ART

Hemophilia A is a congenital hemorrhagic disorder caused by dysfunction or deficiency of coagulation Factor VIII (FVIII). The principle of hemostatic treatment for hemophilia A is regular replacement therapy with coagulation Factor VIII preparations, as a result, the onset of hemophilic arthropathy has been markedly suppressed, greatly contributing to improvement of quality of life. However, even with this standard therapy, there are problems such as the occurrence of alloantibodies (inhibitors) induced by administration of the preparation. In this context, an FIXa/FX bispecific antibody, which is an FVIII-mimetic antibody, has been developed (Patent Document 1). This antibody, which exerts a long-acting effect by subcutaneous administration, exhibits remarkable hemostatic efficacy and is used as regular prophylactic administration for a congenital hemophilia A patient with or without an inhibitor. On the other hand, there remain many clinical issues to be solved, such as thromboembolic events associated with combination with bypassing agents, hemostasis monitoring, perioperative hemostatic management, and hemostatic efficacy at high activity levels.

The FIXalFX bispecific antibody has obtained an indication as a regular administration preparation for a hemophilia A patient, and in cases where bleeding occurs during regular administration, a separate hemostatic agent may be administered. As such a hemostatic agent, in a hemophilia A patient without an inhibitor, an FVIII preparation is the first choice; however, it involves a risk of inducing FVIII inhibitors. In hemophilia A patient with an inhibitor, a recombinant activated blood coagulation Factor VII (rFVIIa) preparation is the first choice as a hemostatic agent; however, since rFVIIa has a relatively short half-life of about 2 to 3 hours, frequent administration is required, which imposes a burden on patients and healthcare professionals. A method of using a Factor IX preparation as a hemostatic agent is also known (Patent Document 2); however, as compared with using a Factor X preparation, it has the problem of weaker effect in promoting thrombin generation.

Non-Patent Literature 2 discloses a method of combining the FIXa/FX bispecific antibody with dried concentrated human blood coagulation Factor X-added activated Factor VII in hemophilia A with an inhibitor. Activated FVII (FVIIa) is a risk factor for FVIIa-related thrombotic complications (Non-Patent Literature 3). Furthermore, since the package insert of dried concentrated human blood coagulation Factor X-added activated Factor VII includes a description in the warning section regarding concomitant use with the FIXa/FX bispecific antibody, there are also problems in terms of safety concerning such combination.

### PRIOR ART DOCUMENTS

### PATENT DOCUMENTS

Patent Document 1: Japanese Patent No.6013915
Patent Document 2: Japanese Patent No. 6698102

### NON-PATENT LITERATURE

Non-Patent Literature 1: Ferriere S, et al. Blood. (2020) 136:740-8
Non-Patent Literature 2: Yuto Nakajima,et al.Haemophilia (2022;28:e149-e152)
Non-Patent Literature 3: Laura Downey,et al.Anesth Analg. (2017;125:1431-1436)

### SUMMARY OF INVENTION

### PROBLEMS TO BE SOLVED BY THE INVENTION

The present invention provides a pharmaceutical composition comprising blood coagulation Factor X for treating a subject with hemophilia A, wherein the blood coagulation Factor X is used in combination with a bispecific antibody that binds to activated blood coagulation Factor IX and blood coagulation Factor X.

### MEANS FOR SOLVING THE PROBLEMS

Accordingly, the present disclosure includes the following:
Item 1. A pharmaceutical composition comprising blood coagulation Factor X for treating a subject with hemophilia A, wherein the blood coagulation Factor X is used in combination with a bispecific antibody that binds to activated blood coagulation Factor IX and blood coagulation Factor X.
Item 2. The pharmaceutical composition of Item 1, wherein the FIXalFX bispecific antibody is administered prior to, simultaneously with, or subsequent to administration of FX.
Item 3. The pharmaceutical composition of Item 1 or 2, wherein the blood coagulation Factor X is administered at 50 to 1000 IU per kg body weight.

### EFFECTS OF THE INVENTION

According to the present disclosure, there is provided a pharmaceutical composition comprising blood coagulation Factor X for treating a subject with hemophilia A, wherein the blood coagulation Factor X is used in combination with a bispecific antibody that binds to activated blood coagulation Factor IX and blood coagulation Factor X.

### BRIEF DESCRIPTION OF DRAWINGS

Figure 1
   Additive effect of Factor X on thrombin generation in coagulation Factor VIII deficient plasma samples in a disease model characterized by deficiency or dysfunction of coagulation Factor VIII. x-axis: Time (minutes). y-axis: Thrombin (nM).
Figure 2
   Additive effect of the FIXalFX bispecific antibody and Factor X on thrombin generation in coagulation Factor VIII deficient plasma samples in a disease model characterized by deficiency or dysfunction of coagulation Factor VIII. x-axis: Time (minutes). y-axis: Thrombin (nM).
Figure 3
   Additive effect of the FIXa/FX bispecific antibody with Factor X (A) or Factor IX (B) on thrombin generation in coagulation Factor VIII deficient plasma samples in a disease model characterized by deficiency or dysfunction of coagulation Factor VIII. x-axis: Time (minutes). y-axis: Thrombin (nM).
Figure 4
   Additive effect of the FIXa/FX bispecific antibody and Factor X on clot formation in coagulation Factor VIII deficient whole blood samples in a disease model characterized by deficiency or dysfunction of coagulation Factor VIII (ROTEM).x-axis: Time (minutes). y-axis: Amplitude (mm). The waveform opened earlier and larger in a concentration-dependent manner of Factor X.
Figure 5
   Additive effect of the FIXa/FX bispecific antibody and Factor X on coagulation time in whole blood samples from mice in which the coagulation Factor VIII gene is knocked out in a disease model characterized by deficiency or dysfunction of coagulation Factor VIII. y-axis: CT+CFT (sec). The coagulation time of mice in which the coagulation Factor VIII gene was knocked out was prolonged about eight-fold compared with wild-type mice. Even when the FIXa/FX bispecific antibody was injected into the mice in which the coagulation Factor VIII gene was knocked out, no change was observed in coagulation time. This is because the antibody does not exhibit cross-reactivity with mice. However, when the antibody and Factor X were injected into the mice, the coagulation time was significantly shortened as compared with that in the non-injection state.
Figure 6
   Additive effect of the FIXa/FX bispecific antibody and Factor X on the fibrin generation rate in whole blood samples from mice in which the coagulation Factor VIII gene is knocked out in a disease model characterized by deficiency or dysfunction of coagulation Factor VIII. y-axis: α(°). The fibrin generation rate of mice in which the coagulation Factor VIII gene was knocked out decreased about three-fold compared with wild-type mice. However, when the antibody and Factor X were injected into the mice in which the coagulation Factor VIII gene was knocked out, the fibrin generation rate was significantly increased as compared with that in the non-injection state.
Figure 7
   Additive effect of the FIXa/FX bispecific antibody and Factor X on bleeding amount from the tail of mice in which the coagulation Factor VIII gene is knocked out in a disease model characterized by deficiency or dysfunction of coagulation Factor VIII. y-axis: Bleeding amount (10 minutes) (µL). The bleeding amount of mice in which the coagulation Factor VIII gene was knocked out increased about 25-fold compared with wild-type mice. However, when the antibody and Factor X were injected into the mice in which the coagulation Factor VIII gene was knocked out, the bleeding amount was significantly decreased as compared with that in the non-injection state.

### MODES FOR CARRYING OUT THE INVENTION

### DETAILED DESCRIPTION OF THE INVENTION

Hereinafter, modes for carrying out the present invention will be described in detail. However, the present invention is not limited to the embodiments described below.

Hemophilia A is a disease in which blood coagulation Factor VIII is deficient or has reduced activity. For example, testing for hemophilia A may be carried out by examining the activity of a coagulation factor. In one embodiment, when the activity of blood coagulation Factor VIII, relative to 100% activity of Factor VIII in plasma of a normal human, is less than 1%, less than 5%, less than 10%, less than 15%, less than 20%, less than 25%, less than 30%, less than 35%, or less than 40%, the subject is diagnosed as having hemophilia A. The activity of Factor VIII is measured, for example, by a one-stage clotting assay or a synthetic substrate method.

In the present specification, the term "treating" is meant to encompass alleviating, suppressing, or preventing a disorder, disease, or condition, or one or more symptoms associated with the disorder, disease, or condition; or mitigating or eliminating the cause of the disorder, disease, or condition itself. For example, in the present specification, treating also includes improvement of the bleeding state in a patient who cannot achieve hemostasis, who requires a long time for hemostasis, or who has difficulty in achieving hemostasis.

In the present specification, the terms "blood coagulation factor," "coagulation factor," or "(blood) coagulation factor," or the abbreviated form "F" preceding each blood coagulation factor number (for example, FVIII, FIX, FX, etc.) are used synonymously, and refer to each human blood coagulation factor of the human coagulation system. Activated coagulation factors are, for example, abbreviated as Factor VIIIa, Factor IXa, Factor Xa, and the like. Non-activated coagulation factors are abbreviated as Factor VIII, Factor IX, Factor X, and the like.

In the present specification, the term "antibody" includes a monoclonal antibody, a polyclonal antibody, an antibody variant (such as a chimeric antibody, a humanized antibody, or a low-molecular-weight antibody including an antibody fragment to which another molecule may optionally be added, a multispecific antibody, and the like), as long as it exhibits desired antigen-binding activity and biological activity. For example, as an "antibody" in the present specification, a molecule in which an HAS-binding scaffold is added to a Fab (only the Fab portion being derived from a normal antibody) is also included. In the present specification, the "antibody" may also be either a polypeptide or a heteromultimer. The antibody may be a monoclonal antibody, a chimeric antibody, a humanized antibody, a human antibody, an Fc-fusion antibody, or a low-molecular-weight antibody such as an antibody fragment. In the present specification, the term "antibody" refers to a binding protein including an antigen binding site. The terms "binding site" or "antigen binding site" as used in the present specification denote the region of an antibody molecule to which the antigen actually binds. The term "antigen binding site" includes an antibody heavy chain variable domain (VH) and an antibody light chain variable domain (VL) (a VH/VL pair).

In the present specification, the term "antibody fragment" refers to a molecule other than a full-length antibody that includes a portion of the full-length antibody which binds to an antigen to which the full-length antibody binds. Examples of the antibody fragment include, but are not limited to, an Fv, a Fab, a Fab', a Fab'-SH, an F(ab')₂, a diabody, a linear antibody, a single-chain antibody molecule (for example, an scFv), and a multispecific antibody formed from antibody fragments.

In the present specification, the term "chimeric antibody" refers to an antibody in which a portion of a heavy chain and/or a light chain is derived from a particular source or species, while the remaining portion of the heavy chain and/or the light chain is derived from a different source or species.

In the present specification, the "class" of an antibody refers to the type of constant domain or constant region present in the heavy chain of the antibody. There are five major classes of antibodies: IgA, IgD, IgE, IgG, and IgM. Some of these may further be divided into subclasses (isotypes), for example, IgG1, IgG2, IgG3, IgG4, IgA1, and IgA2. The heavy chain constant domains corresponding to the different classes of immunoglobulins are referred to as α, δ, ε, γ, and µ, respectively.

Factor X is one of the enzymes constituting the blood coagulation cascade. Factor X is generally activated by intrinsic Xase or extrinsic Xase to become Factor Xa. Factor Xa generally acts by cleaving prothrombin at two sites (the Arg-Thr bond and subsequently the Arg-Ile bond) to produce active thrombin.

In one embodiment, there is provided a pharmaceutical composition comprising blood coagulation Factor X for use in treating a subject with hemophilia A, wherein the blood coagulation Factor X is used in combination with a bispecific antibody that binds to activated blood coagulation Factor IX and blood coagulation Factor X. In another embodiment, there is provided blood coagulation Factor X for use in treating a subject with hemophilia A, wherein the blood coagulation Factor X is used in combination with a bispecific antibody that binds to activated blood coagulation Factor IX and blood coagulation Factor X. In a further embodiment, there is provided a combination of blood coagulation Factor X and a bispecific antibody that binds to activated blood coagulation Factor IX and blood coagulation Factor X for use in treating a subject with hemophilia A. In another embodiment, there is provided the use of blood coagulation Factor X for the manufacture of a medicament for treating a subject with hemophilia A, wherein the treating comprises combining blood coagulation Factor X with a bispecific antibody that binds to activated blood coagulation Factor IX and blood coagulation Factor X. In a further embodiment, there is provided a method for treating a subject with hemophilia A, comprising a use of a combination of blood coagulation Factor X and a bispecific antibody that binds to activated blood coagulation Factor IX and blood coagulation Factor X.

In one embodiment, the bispecific antibody that binds to activated blood coagulation Factor IX and blood coagulation Factor X is referred to as an FIXa/FX bispecific antibody, and includes a first antigen binding site and a second antigen binding site, and these sites are not particularly limited as long as they show binding activity to FIXa and FX, respectively. The FIXalFX bispecific antibody of the present invention may further bind to another antigen as long as it has binding activity to FIXa and FX. In the present specification, the term "bispecific antibody that binds to FIXa and FX" refers to a bispecific antibody that can bind to FIXa and FX with sufficient affinity such that the antibody is useful as a therapeutic agent when it targets FIXa and FX. In one aspect, the extent of binding of the bispecific antibody that binds to FIXa and FX to an unrelated non-FIX protein, non-FIXa protein, or non-FX protein is less than about 10% of the binding of the antibody to FIXa and FX when measured, for example, by a radioimmunoassay (RIA). In a particular aspect, an antibody that binds to FIXa and FX has a dissociation constant (Kd) of ≤100 µM, ≤10 µM, ≤1 µM, ≤100 nM, ≤10 nM, ≤1 nM, ≤0.1 nM, ≤0.01 nM, or ≤0.001 nM (for example, less than or equal to 10⁻⁵ M, for example, from 10⁻⁵ M to 10⁻¹⁰ M, or for example, from 10⁻⁶ M to 10⁻¹⁰ M). In a particular aspect, the bispecific antibody that binds to FIXa and FX binds to an epitope of FIX, FIXa, and FX that are conserved among FIX, FIXa, and FX from different species, respectively. For example, ACE910 (Emicizumab) is exemplified as an FIXalFX bispecific antibody (Patent Document 1). In one embodiment, an antibody that binds to FIXa and FX has a dissociation constant (Kd) of ≤1 µM in a surface plasmon resonance assay.

In one embodiment, hemophilia A may be a congenital or an acquired disease. In another embodiment, a patient with hemophilia A has a deficiency of coagulation Factor VIII or suffers from a dysfunction of coagulation Factor VIII.

In one embodiment of the present invention, the components of the combination, the FIXa/FX bispecific antibody and FX, may be formulated together or may be formulated separately. When formulated separately, administration of FX and administration of the FIXa/FX bispecific antibody may be carried out simultaneously or sequentially. For example, administration of the FIXa/FX bispecific antibody may be carried out prior to, simultaneously with, or subsequent to administration of FX. In one aspect, the component FIXa/FX bispecific antibody of the combination may independently be administered as a single dose or as multiple doses. When administered as multiple doses, for example, the dosing interval of the FIXa/FX bispecific antibody is within about 1 month, 2 months, 3 months, 4 months, 5 months, or 6 months, or within about 1, 2, or 3 weeks, or within about 1, 2, 3, 4, 5, or 6 days, and the dosing interval of FX is about 12, 14, 16, 18, 20, 22, 24, 26, 28, 30, 32, 34, 36, 38, 40, 42, 44, 46, or 48 hours.

The FIXa/FX bispecific antibody or FX of the present invention may be administered by any suitable means, including parenteral administration, pulmonary administration, and intranasal administration, and, when desired for local treatment, intralesional administration. Parenteral administration includes intramuscular, intravenous, intra-arterial, intraperitoneal, or subcutaneous administration. Administration may be carried out by any suitable route, depending in part on whether the administration is short-term or long-term, for example, by injection such as intravenous injection or subcutaneous injection. Various administration schedules are included, including, but not limited to, single administration, repeated administration over various time points, bolus administration, and pulse infusion. Preferably, the FIXa/FX bispecific antibody is injected subcutaneously, and FX is injected intravenously.

The FIXa/FX bispecific antibody is administered, for example, to an adult at about 0.5 mg/kg body weight, about 1 mg/kg body weight, about 1.5 mg/kg body weight, about 2 mg/kg body weight, about 2.5 mg/kg body weight, about 3 mg/kg body weight, about 3.5 mg/kg body weight, about 4 mg/kg body weight, about 4.5 mg/kg body weight, about 5 mg/kg body weight, about 5.5 mg/kg body weight, about 6 mg/kg body weight, about 6.5 mg/kg body weight, about 7 mg/kg body weight, about 7.5 mg/kg body weight, about 8 mg/kg body weight, about 8.5 mg/kg body weight, about 9 mg/kg body weight, about 9.5 mg/kg body weight, about 10 mg/kg body weight, or more per administration. Preferably, the FIXa/FX bispecific antibody is administered at about 1.5 mg/kg body weight, about 3 mg/kg body weight, or about 6 mg/kg body weight.

The blood coagulation Factor X is administered, for example, to an adult at about 50 IU/kg body weight, about 75 IU/kg body weight, about 100 IU/kg body weight, about 150 IU/kg body weight, about 200 IU/kg body weight, about 250 IU/kg body weight, about 300 IU/kg body weight, about 350 IU/kg body weight, about 400 IU/kg body weight, about 450 IU/kg body weight, about 500 IU/kg body weight, about 550 IU/kg body weight, about 600 IU/kg body weight, about 650 IU/kg body weight, about 700 IU/kg body weight, about 750 IU/kg body weight, about 800 IU/kg body weight, about 850 IU/kg body weight, about 900 IU/kg body weight, about 950 IU/kg body weight, about 1000 IU/kg body weight, or more per administration. Preferably, the blood coagulation Factor X is administered at 50 to 1000 IU/kg body weight, and more preferably at about 200 IU/kg body weight or more.

The blood coagulation factor used in the present invention may be a natural protein derived from a living body or may be a recombinant protein. When derived from a living body, it may be derived from plasma. The plasma is preferably human plasma.

In the present specification, the term "pharmaceutical composition" refers to a preparation in a form such that the biological activity of the active ingredient contained therein can exert its effect. The term "pharmaceutically acceptable carrier" refers to a component other than the active ingredient in the pharmaceutical composition that is nontoxic to the subject. The pharmaceutically acceptable carrier includes, but is not limited to, a buffer, an excipient, a stabilizer, or a preservative.

In one embodiment of the present invention, by combining coagulation Factor X with an FIXa/FX bispecific antibody, blood coagulation activity is improved as compared with a treatment using the FIXa/FX bispecific antibody without combination with Factor X. In another embodiment of the present invention, the blood coagulation activity of the treatment is improved in a dose-dependent manner of Factor X. For example, blood coagulation activity can be examined by measuring thrombin amount, thrombin generation rate, blood coagulation time, clot formation, fibrin formation, fibrin formation rate, bleeding frequency, or bleeding amount. For example, when thrombin generation amount is increased, thrombin generation rate is elevated, blood coagulation time is shortened, clot formation is enhanced, bleeding frequency is reduced, and/or bleeding amount is decreased, blood coagulation activity is considered to be improved. In the present specification, for example, when, by treatment, a patient with hemophilia A reaches an equivalent of 1%, 2.5%, 5%, 10%, 15%, 20%, or 25% or more in terms of Factor VIII activity, preferably 5% or more, blood coagulation activity is considered to be improved (with the Factor VIII activity of a normal human set as 100%).

In one embodiment of the present invention, by combining coagulation Factor X with an FIXa/FX bispecific antibody, thrombin generation may be increased in a patient. Thrombin is an important enzyme in coagulation, and it is known that enhancing the production of thrombin exerts various effects on the coagulation system. Thrombin generally converts fibrinogen into fibrin, thereby forming fibrin, and thrombin activates platelets. An increase in thrombin generation may also cause an increase in fibrin formation and activation of platelets. Furthermore, regular production of thrombin may also cause a decrease in fibrinolytic activity. For example, thrombin generation in biological plasma can be measured using the calibrated automated thrombogram (CAT) method (Thermo Fisher Scientific).

In one embodiment of the present invention, since administration of Factor X, which is a non-activated coagulation factor, is intended, the risk of thrombosis is expected to be lower as compared with the application of an activated prothrombin complex concentrate comprising an activated coagulation factor, dried concentrated human blood coagulation Factor X-added activated Factor VII, or Factor VIIa. Since no Factor VIII-related factor is present, application to a patient with hemophilia A is expected to have little or no risk of inducing an inhibitor against Factor VIII.

The combination of the present invention is used in advance when bleeding occurs or when bleeding is expected, although the period is not particularly limited. For example, the combination of the present invention may be used at the time of bleeding after trauma, during surgery, or before surgery.

In one embodiment of the present invention, since the half-life of Factor X (40 hours) is longer than the half-life of activated Factor VII (2.5 hours), the combination with the FIXa/FX bispecific antibody may reduce the risk of inhibitor development in a hemophilia A patient with an inhibitor. In a hemophilia A patient with an inhibitor, as compared with activated Factor VII, the combination may also reduce the burden of frequent injections by maintaining a longer dosing interval.

In one embodiment of the present invention, unlike Non-Patent Literature 2, FVIIa is not used, and since FX, which is a non-activated coagulation factor, is used in combination with a bispecific antibody that binds to activated blood coagulation Factor IX and blood coagulation Factor X, the invention is advantageous in terms of safety, such as reduced risk of hypercoagulation.

In one embodiment of the present invention, since non-activated Factor X has a relatively short half-life (40 hours) as compared with the half-life of the FIXalFX bispecific antibody (about 4 to 5 weeks after subcutaneous injection), the effect of promoting thrombin generation may be limited to a certain period, such as during hemostasis, and therefore may reduce the risk of thrombosis during long-term treatment.

In one embodiment of the present invention, the subject to be treated is not particularly limited, but is preferably a human. In another embodiment, the subject to be treated either has developed an inhibitor against FVIII or FVIIIa (sometimes referred to as with an inhibitor), or has not developed such an inhibitor (sometimes referred to as without an inhibitor). According to the combination of the present invention, administration of FVIII or FVIIIa can be reduced or avoided.

In one embodiment of the present invention, the subject to be treated may have received treatment with the FIXalFX bispecific antibody prior to administration of the combination. The treatment with the FIXalFX bispecific antibody prior to administration of the combination is not particularly limited, but may be received temporarily or regularly. Preferably, the treatment is received regularly.

In the present specification, the term "about" refers to a range of ±10%, preferably ±5%, and more preferably ±2.5%.

The following Examples are provided to illustrate the present invention specifically and in detail, but the Examples are used for exemplification of the present invention and are not intended to limit the present invention.

### EXAMPLES

### Experimental Procedure:

### Example 1

Thrombin Generation in Factor VIII-Deficient Plasma Factor VIII deficient plasma samples (Factor VIII-deficient plasma, Affinity Biologicals Inc.) were used as a model of deficiency or dysfunction of coagulation Factor VIII.

The FIXa/FX bispecific antibody (hereinafter abbreviated as "Emi"; details are described in Patent Document 1) was added to the plasma sample at a concentration of 50 µg/ml. Furthermore, Factor X was added in vitro to part of the experiment. Plasma derived from healthy humans was used as a control in the experiment.

**Table 1: Combinations of Tested Additives**

| | | |
|---|---|---|
| FVIII dp | | |
| FVIII dp | +130 nM FX | |
| FVIII dp | +260 nM FX | |
| FVIII dp | +520 nM FX | |
| FVIII dp | +780 nM FX | |
| FVIII dp | +1040 nM FX | |
| FVIII dp | +130 nM FX | +50 *µ*g/ml Emi |
| FVIII dp | +260 nM FX | +50 *µ*g/ml Emi |
| FVIII dp | +520 nM FX | +50 *µ*g/ml Emi |
| FVIII dp | +780 nM FX | +50 *µ*g/ml Emi |
| FVIII dp | +1040 nM FX | +50 *µ*g/ml Emi |
| FVIII dp | +125 nM FX | +50 *µ*g/ml Emi |
| FVIII dp | +250 nM FX | +50 *µ*g/ml Emi |
| FVIII dp | +500 nM FX | +50 *µ*g/ml Emi |
| FVIII dp | +125 nM FIX | +50 *µ*g/ml Emi |
| FVIII dp | +250 nM FIX | +50 *µ*g/ml Emi |
| FVIII dp | +500 nM FIX | +50 *µ*g/ml Emi |
| Normal plasma | | |

| | | |
|---|---|---|
| FVIII dp: FVIII -deficient plasma | | |

Thrombin generation was continuously determined using a fluorogenic substrate after coagulation was activated with a small amount of tissue factor (the device and all reagents were from Thermo Fisher Scientific).

### Method for Measuring Thrombin Generation:

Thrombin generation in biological plasma was measured using the calibrated automated thrombogram (CAT) method (Thermo Fisher Scientific). Briefly, thrombin generation is triggered via the extrinsic coagulation pathway by addition of 1 pM tissue factor (TF), phospholipid, and calcium ions (Ca²⁺). A low-affinity fluorogenic substrate is added for real-time analysis of thrombin generation. By calibrating the plasma sample against a known thrombin calibrator, corrections are made for substrate consumption, sample color, and inner filter effects. Fluorescence is detected using the Thermo Fluoroskan. Thrombin activity is calculated from the measured fluorescence signal. The resulting curve shows free thrombin activity (y-axis, thrombin in nM) as a function of time (x-axis, seconds).

### Control Measurement: Normal Plasma and Factor VIII-Deficient Plasma

Analysis of Factor VIII-deficient plasma alone, as expected, showed very weak thrombin generation. This indicates the physiological reason for the bleeding disorder in a patient with hemophilia A.

### Addition of Factor X

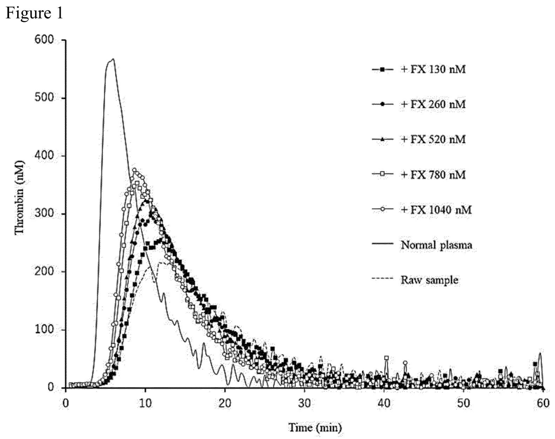

By addition of Factor X, thrombin was rapidly generated, and a total of about 1.5 times more thrombin was produced compared with FVIII deficient plasma samples (Figure 1).

### Addition of Emi

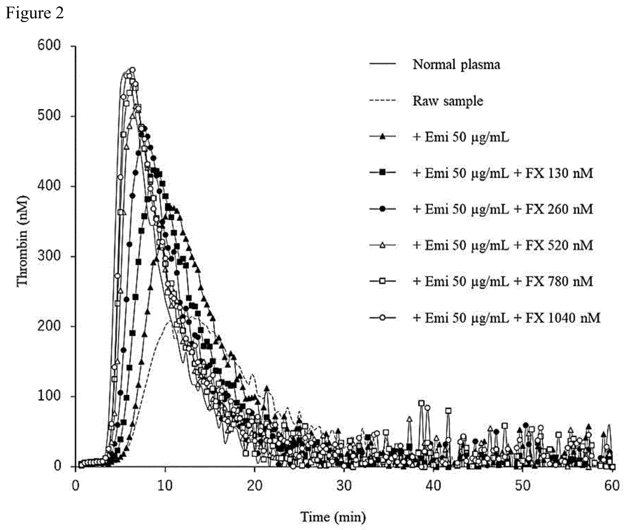

By addition of Emi and Factor X, an increase in thrombin generation comparable to that of Factor VIII deficient plasma samples was obtained (Figure 2).

### Comparison of the Activity of Combination of Emi and Factor X with the Activity of Factor X in Thrombin Generation of Factor VIII-Deficient Plasma Samples:

When thrombin generation in samples supplemented with the combination of Emi and Factor X or with Factor X alone was compared, the combination of Emi and Factor X produced more thrombin, and the time to the thrombin generation peak was shorter with the combination of Emi and Factor X than with Factor X alone (Figures 1 and 2).

### Addition of Factor X or Factor IX

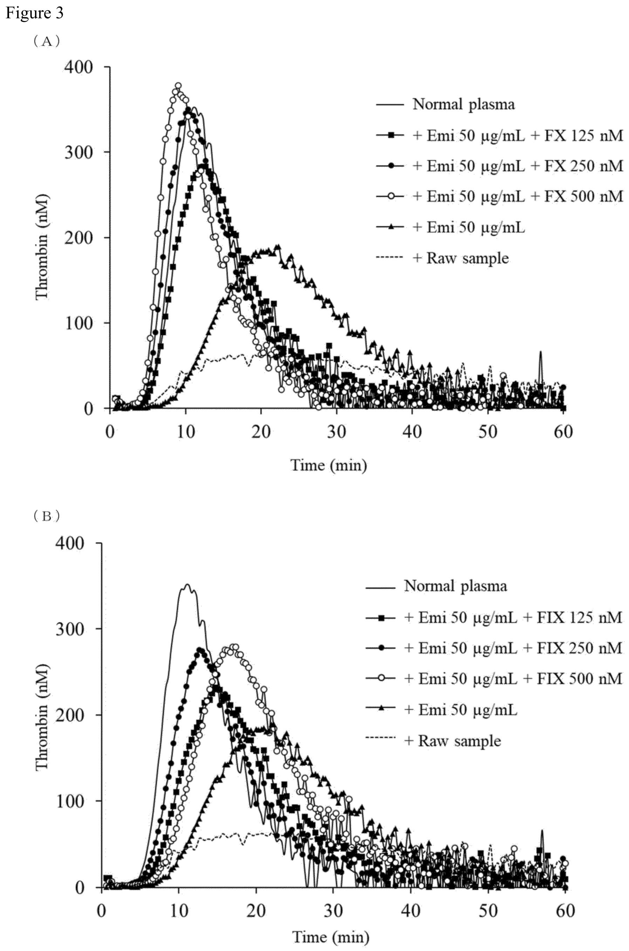

When Factor IX was added to Emi, recovery of thrombin generation ability to the normal plasma level was not observed (Figure 3B). On the other hand, when Factor X was added to Emi, thrombin generation ability was recovered to a level comparable to that of normal plasma (Figure 3A).

### Example 2

### Clot Formation in Whole Blood of Factor VIII-Deficient Model Mice

Hemophilia A model mice in which the gene (F8) encoding Factor VIII on the sex chromosome was knocked out (containing normal levels of non-activated Factor IX, Factor X, and Factor II) were used as a model animal of deficiency or dysfunction of coagulation Factor VIII.

It is known that Emi does not show cross-reactivity with mouse-derived Factor IX and mouse-derived Factor X. In order to evaluate the combined effect of Emi and Factor X in hemophilia A model mice, human-derived Factor IX and human-derived Factor X were administered in addition to Emi, based on Non-Patent Literature 1. Wild-type mice were used as controls in the experiment.

**Table 2: Combinations of Administered Tested Substances**

| HA model mouse | | | |
|---|---|---|---|
| HA model mouse | +3 mg/kg body weight Emi | | |
| HA model mouse | | +100 IU/kg hFIX | +100 IU/kg hFX |
| HA model mouse | +3 mg/kg body weight Emi | +100 IU/kg hFIX | +100 IU/kg hFX |
| HA model mouse | +3 mg/kg body weight Emi | +100 IU/kg hFIX | +200 IU/kg hFX |
| HA model mouse | +3 mg/kg body weight Emi | +100 IU/kg hFIX | +500 IU/kg hFX |
| Normal mouse | | | |

| | | | |
|---|---|---|---|
| HA model mouse: Hemophilia A model mouse hFIX: human Factor IX hFX: human Factor X | | | |

### Rotational Thromboelastometry (ROTEM):

ROTEM was continuously measured for the clot formation process after coagulation was activated with calcium chloride (the device and all reagents were from Pentapharm, Germany).

Clot formation in whole blood was measured using a whole blood hemostasis analyzer (Pentapharm, Germany). Briefly, the measuring system of the device employs a fixed cylindrical cup and a permanently oscillating vertical axis. The movement of the axis is detected by an optical detection system and processed and analyzed by a computer equipped with dedicated software. A test solution is placed into the cylindrical cup, and the cup is posfitioned so that the tip of a pin attached to the lower end of the vertical axis is immersed in the test solution. The center of the axis is guided by a bearing, and the axis oscillates laterally at a constant angle through a spring connector. The rotation of the axis is optically measured via a mirror plate at the upper end of the axis, a diode as a light source, and a photosensitive sensor. When coagulation does not occur, the movement is not impeded, but when a clot is formed and adheres to the surface of the pin and the cup, the movement is impeded. The resulting curve shows clot strength (y-axis, mm) as a function of time (x-axis, seconds).

### Control Measurement: Hemophilia A Model Mice and Wild-Type Mice

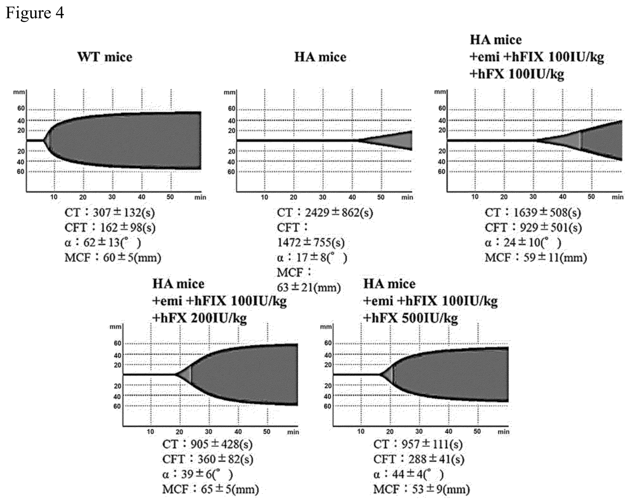

Analysis of hemophilia A model mice, as expected, revealed very weak clot formation. This indicates the physiological reason for the bleeding disorder in a patient with hemophilia A. In addition, analysis of wild-type mice, as expected, showed very strong clot formation compared with hemophilia A model mice (Figure 4).

### Administration of Emi, Human-Derived Factor IX, and Human-Derived Factor X

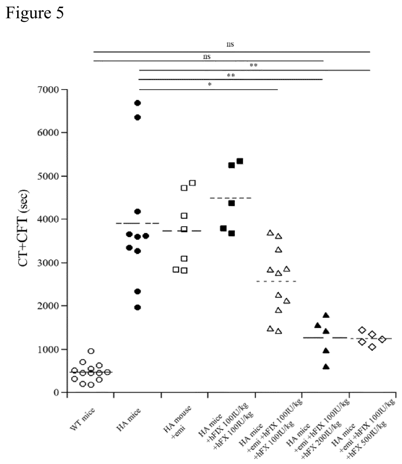

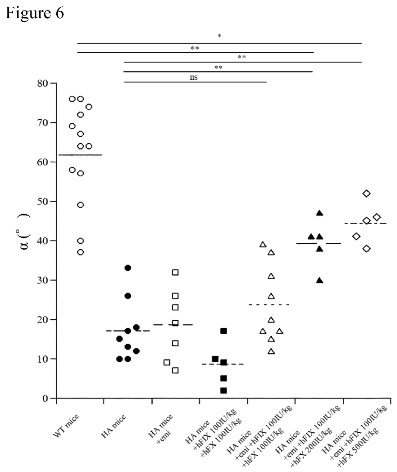

By administration of Emi, human-derived Factor IX, and human-derived Factor X, clot formation was rapidly generated, and compared with FVIII deficient plasma samples, clot formation was generated at up to about 2.5 times the rate (Figures 4, 5, and 6). In addition, the effect was dose-dependent on Factor X (Figures 5 and 6).

### Example 3

### Tail Clip Assay in Factor VIII-Deficient Model Mice

Hemophilia A model mice in which the gene (F8) encoding Factor VIII on the sex chromosome was knocked out (containing normal levels of non-activated Factor IX, Factor X, and Factor II) were used as a model animal of deficiency or dysfunction of coagulation Factor VIII.

Emi was administered to the hemophilia A model mouse at a concentration of 3 mg/kg (50 µg/ml), which is similar to a clinically applied concentration of Emi.

It is known that Emi does not show cross-reactivity with mouse-derived Factor IX and mouse-derived Factor X. In order to evaluate the combined effect of Emi and Factor X in hemophilia A model mice, human-derived Factor IX and human-derived Factor X were administered in addition to Emi, based on Non-Patent Literature 1. Wild-type mice were used as controls in the experiment.

**Table 3: Combinations of Administered Tested Substances**

| HA model mouse | | | |
|---|---|---|---|
| HA model mouse | +3 mg/kg body weight Emi | | |
| HA model mouse | | +100 IU/kg hFIX | +100 IU/kg hFX |
| HA model mouse | +3 mg/kg body weight Emi | +100 IU/kg hFIX | +100 IU/kg hFX |
| HA model mouse | +3 mg/kg body weight Emi | +100 IU/kg hFIX | +200 IU/kg hFX |
| HA model mouse | +3 mg/kg body weight Emi | +100 IU/kg hFIX | +500 IU/kg hFX |
| Normal mouse | | | |

| | | | |
|---|---|---|---|
| HA model mouse: Hemophilia A model mouse hFIX: human-derived Factor IX hFX: human-derived Factor X | | | |

### Tail Clip Assay:

In the tail clip assay, 3 mg/kg (50 µg/ml) of Emi was administered to hemophilia A model mice under anesthesia 24 hours before tail clipping, and human-derived Factor IX and human-derived Factor X were administered 5 minutes before tail clipping. The tail was cut at a position 5 mm from the tip, immediately immersed in a tube containing physiological saline, and the bleeding amount (µL) over 10 minutes was measured.

### Control Measurement: Hemophilia A Model Mice and Wild-Type Mice

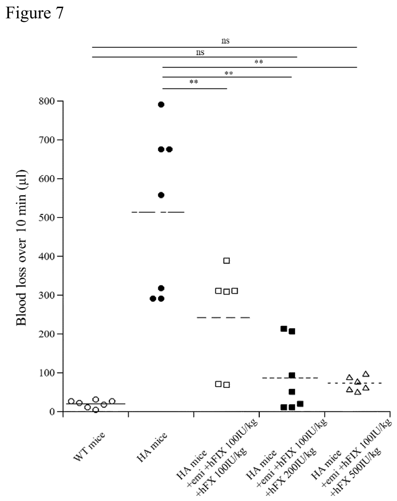

Analysis of hemophilia A model mice, as expected, revealed a very large bleeding amount. This indicates the physiological reason for the bleeding disorder in a patient with hemophilia A. In addition, analysis of wild-type mice, as expected, showed a very small bleeding amount compared with hemophilia A model mice (Figure 7).

### Administration of Emi, Human-Derived Factor IX, and Human-Derived Factor X

By administration of Emi, human-derived Factor IX, and human-derived Factor X, the bleeding amount was reduced, and the effect was dose-dependent on Factor X. In addition, compared with Factor VIII deficient samples, the bleeding amount was reduced by up to about fivefold (Figure 7).

### Industrial Applicability

A pharmaceutical composition comprising blood coagulation Factor X for treating a subject with hemophilia A is provided.

## Claims

1. A pharmaceutical composition comprising blood coagulation Factor X for treating a subject with hemophilia A, wherein the blood coagulation Factor X is used in combination with a bispecific antibody that binds to activated blood coagulation Factor IX and blood coagulation Factor X.

2. The pharmaceutical composition of claim 1, wherein the FIXalFX bispecific antibody is administered prior to, simultaneously with, or subsequent to administration of FX.

3. The pharmaceutical composition of claim 1 or 2, wherein the blood coagulation Factor X is administered at 50 to 1000 IU per kg body weight.
